Europäisches Patentamt

European Patent Office

Office européen des brevets

(19)

(11) **EP 1 009 370 B1**

(12) **EUROPÄISCHE PATENTSCHRIFT**

(45) Veröffentlichungstag und Bekanntmachung des Hinweises auf die Patenterteilung:
**27.02.2002 Patentblatt 2002/09**

(21) Anmeldenummer: **98945310.5**

(22) Anmeldetag: **05.09.1998**

(51) Int Cl.⁷: **A61K 7/16**

(86) Internationale Anmeldenummer:
**PCT/EP98/05632**

(87) Internationale Veröffentlichungsnummer:
**WO 99/13851 (25.03.1999 Gazette 1999/12)**

(54) **ZAHNREINIGUNGSMITTEL MIT EINER POLIERMITTELKOMBINATION AUS KIESELSÄUREPOLIERMITTELN UND ALUMINIUMOXID**

CLEANING AGENT FOR DENTAL USE COMPRISING A COMBINATION OF POLISHING AGENTS BASED ON A SILICIC ACID AND ALUMINIUM OXIDE

AGENT NETTOYANT A USAGE DENTAIRE COMPRENANT UNE COMBINAISON D'AGENTS POLISSANTS A BASE D'ACIDE SILICIQUE ET D'OXYDE D'ALUMINIUM

(84) Benannte Vertragsstaaten:
**AT BE CH CY DE DK ES FI FR GB GR IT LI NL PT SE**

(30) Priorität: **15.09.1997 DE 19740453**

(43) Veröffentlichungstag der Anmeldung:
**21.06.2000 Patentblatt 2000/25**

(73) Patentinhaber: **Henkel Kommanditgesellschaft auf Aktien**
**40589 Düsseldorf-Holthausen (DE)**

(72) Erfinder:
• **LEINEN, Hans-Theo**
 **D-40229 Düsseldorf (DE)**
• **WÜLKNITZ, Peter**
 **D-42799 Leichlingen (DE)**

(56) Entgegenhaltungen:
**DE-A- 2 758 548       DE-A- 3 425 152**
**GB-A- 2 230 189       US-A- 5 094 844**
**US-A- 5 605 677**

**Beschreibung**

Die Erfindung betrifft ein Zahnreinigungsmittel in Form einer wäßrigen, pastösen oder flüssigen Creme mit einem Gehalt an 10 - 30 Gew.-% einer Poliermittelkombination und 20 - 50 Gew.-% eines Feuchthaltemittels, das durch einen Zusatz an kondensierten Phosphaten in der Reinigungsleistung weiter verbessert wurde.

Zahnpasten werden bei der täglichen Reinigung der Zähne durch Bürsten mit einer Zahnbürste angewendet. Die Zahnpasta soll in erster Linie die Reinigung der Zahnoberflächen von Speiseresten, Verfärbungen durch z.B. Tabak oder Tee und von den fest anhaftenden bakteriellen Zahnbelägen, der sogenannten Plaque, unterstützen. Dies geschieht hauptsächlich durch die in der Zahnpasta enthaltenen Poliermittel, in geringerem Maße auch durch die enthaltenen Tenside. Die Poliermittel müssen, um ihre Reinigungs- und Polierwirkung zu entfalten, eine gewisse Abrasivität gegenüber der Zahnoberfläche aufweisen. Es ist jedoch von größter Bedeutung, daß die Abrasivität gegenüber Zahnschmelz und Dentin auf niedrigen Werten gehalten wird, um eine Schädigung der Zahnoberfläche durch den täglichen Gebrauch der Zahnpasta zu vermeiden. Vor allem dürfen die verwendeten Poliermittel keine tiefen Kratzer auf der Zahnoberfläche verursachen. Erwünscht ist vielmehr eine Einebnung der ggf. vorhandenen Rauhigkeit der Zahnoberfläche.

Aus zahlreichen Druckschriften sind kondensierte Phosphate als Antizahnstein-Wirkstoffe und als Demineralisierungs-Inhibitoren in Zahnpflegemitteln bekannt.

Aus US 4,822,599 A war auch bereits eine reinigende Wirkung der wasserlöslichen Pyrophosphat-Salze für durch Tee und Kaffee verfärbte Zähne bekannt.

Aus DE 27 58 548 C2 waren Zahnpflegemittel mit einem Putzkörpergemisch aus einem Kieselsäurepoliermittel und einem calcinierten Aluminiumoxid als besonders wirksame Zahnreinigungsmittel bekannt. Zur Verminderung des Zahnschmelz-Abriebs wurde dort ein Zusatz von bestimmten anorganischen Elektrolytsalzen empfohlen. Trotzdem wurden nur äußerst hohe Dentinabriebwerte (RDA) und Schmelzabriebwerte (REA) im Bereich von 300 - 500 erreicht.

Durch Reduzierung oder Weglassen des Aluminiumoxid-Poliermittels lassen sich zwar die Abriebwerte senken, gleichzeitig wird aber das Reinigungsvermögen deutlich verschlechtert. Es bestand daher die Aufgabe, ein Zahnreinigungsmittel zu entwickeln, das eine sehr geringe Abrasivität, etwa mit RDA- und REA-Werten unter 100, und trotzdem eine sehr gute Reinigungsleistung, etwa mit CRS-Werten um 100, aufweist.

Aus DE 34 25 152 war eine Poliermittelkombination aus einem Kieselsäure-Poliermittel und einem schwach calcinierten Aluminiumoxid bekannt, mit welcher sich die Abrasivität bei guter Reinigungsleistung deutlich senken ließ.

Es wurde nunmehr gefunden, daß kondensierte Phosphate in idealer Weise dazu geeignet sind, die Reinigungsleistung von Zahnpasten, die eine Poliermittelkombination aus Kieselsäure-Poliermitteln und Alurniniumoxid-Poliermitteln enthalten, so sehr zu steigern, daß die gestellte Aufgabe erfüllt wird, ohne daß die Abriebwerte wesentlich erhöht werden.

Gegenstand der Erfindung sind daher Zahnreinigungsmittel in Form von wäßrigen, pastösen oder flüssigen Dispersionen mit einem Gehalt von 10 - 30 Gew.-% einer Poliermittelkombination aus Kieselsäure-Poliermitteln und Aluminiumoxid im Gewichtsverhältnis 10 : (0,2- 2) und 20 bis 50 Gew.-% eines Feuchthaltemittels aus der Gruppe Sorbit, Glycerin, Propylenglycol-1,2 oder Gemischen davon, die zur Erhöhung der Reinigungswirkung ein kondensiertes Phosphat aus der Gruppe Tripolyphosphat, Pyrophosphat, Trimetaphosphat oder Gemische davon in Form der Alkali- oder Ammoniumsalze in einer Menge von 2 - 12 Gew.-% enthalten.

Geeignete Kieselsäure-Poliermittel sind z.B. Gelkieselsäuren, die durch Umsetzung von Natriumsilikatlösungen mit starken wäßrigen Mineralsäuren unter Ausbildung eines Hydrosols, Alterung zum Hydrogel, Waschen und Trocknen erzeugt werden. Erfolgt die Trocknung unter schonenden Bedingungen auf Wassergehalte von 15 - 35 Gew.-%, so werden sogenannte Hydrogelkieselsäuren erhalten, wie sie z.B. aus US 4,153,680 bekannt sind.

Durch Trocknung auf Wassergehalte von unterhalb 15 Gew.-% erfolgt eine irreversible Schrumpfung der vorher lockeren Struktur des Hydrogels zur dichten Struktur des sogenannten Xerogels. Solche Xerogelkieselsäuren sind z.B. in US 3,538,230 beschrieben.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet sind z.B. Fällungskieselsäuren, die eine mittlere Teilchengröße von 5 - 20 μm, einen Siebrückstand 45μm von weniger als 1 Gew.-% und eine spezifische Oberfläche (BET) von 100 - 300 $m^2$/g aufweisen.

Die erfindungsgemäßen Zahnpflegemittel weisen aufgrund der speziellen Poliermittelkombination ein hervorragendes Reinigungsvermögen auch gegenüber Zahnverfärbungen durch Tee und Nikotin auf. Gleichzeitig wird bei nur mäßigem Dentin- und Schmelzabrieb eine hohe Polierwirkung (Einebnung von Aufrauhungen) erzielt. Trotz des Gehaltes an einer relativ harten Poliermittelkomponente, des Aluminiumoxids, weisen die erfindungsgemäßen Zahnreinigungsmittel praktisch keine Kratzwirkung auf.

Als Aluminiumoxid-Poliermittel eignet sich bevorzugt eine schwach calcinierte Tonerde mit einem Gehalt von wenigstens 10 Gew.-%, von Alpha-Aluminiumoxid verschiedener, sogenannter Gamma-Aluminiumoxid-Modifikationen.

Geeignete schwach calcinierte Tonerden werden durch Calcination aus Aluminiumhydroxid hergestellt. Aluminiumhydroxid geht durch Calcination in das bei Temperaturen oberhalb 1200°C thermodynamisch stabile $\alpha$-$Al_2O_3$ über. Die bei Temperaturen zwischen 400 und 1000°C auftretenden, thermodynamisch instabilen $Al_2O_3$-Modifikationen bezeichnet man als Gamma-Formen (vgl. Ullmann, Enzyklopädie der technischen Chemie, 4. Auflage (1974), Band 7, Seite 298). Durch Wahl der Temperatur und der Zeitdauer bei der Calcination kann man den Calcinationsgrad, d.h. die Umwandlung in das thermodynamisch stabile $\alpha$-$Al_2O_3$ auf beliebige Höhe einstellen. Man erhält durch schwache Calcination eine Tonerde mit einem Gehalt an $\gamma$-$Al_2O_3$, der um so niedriger ist, je höher die Calcinationstemperatur und je länger die Calcinationsdauer gewählt wird. Schwach calcinierte Tonerden unterscheiden sich von reinem $\alpha$-$Al_2O_3$ durch eine geringere Härte der Agglomerate, eine größere spezifische Oberfläche und größere Porenvolumina.

Der Dentinabrieb (RDA) der erfindungsgemäß zu verwendenden schwächer calcinierten Tonerden mit einem Anteil von 10 - 50 Gew.-% $\gamma$-$Al_2O_3$ beträgt nur 30 - 60 % des Dentinabriebs eines stark calcinierten, reinen $\alpha$-$Al_2O_3$ (gemessen in einer Standard-Zahnpaste mit 20 Gew.-% Tonerde als einzigem Poliermittel).

Im Gegensatz zu $\alpha$-$Al_2O_3$ läßt sich das $\gamma$-$Al_2O_3$ mit einer wäßrig-ammoniakalischen Lösung von Alizarin S (1,2-Dihydroxy-9,10-anthrachinon-4-sulfonsäure) rot anfärben. Man kann den Grad der Anfärbbarkeit als Maß für den Calcinationsgrad bzw. für den Anteil an $\delta$-$Al_2O_3$ in einer calcinierten Tonerde wählen :

Ca. 1 g $Al_2O_3$, 10 ml einer Lösung von 2 g/l Alizarin S in Wasser und 3 Tropfen einer wäßrigen, 10 Gew.-%igen Lösung von $NH_3$ werden in ein Reagenzglas gegeben und kurz aufgekocht. Das $Al_2O_3$ wird anschließend abfiltriert, nachgewaschen, getrocknet und unter dem Mikroskop beurteilt oder farbmetrisch ausgewertet.

Geeignete, schwach calcinierte Tonerden mit einem Gehalt von 10 - 50 Gew.-% $\gamma$-$Al_2O_3$ lassen sich nach diesem Verfahren schwach bis tief rosa anfärben.

Aluminiumoxid-Poliermittel verschiedener Calcinationsgrade, Mahlfeinheit und Schüttgewichte sind im Handel erhältlich, z.B. die "Poliertonerden" der Firma Giulini-Chemie beziehungsweise ALCOA.

Eine bevorzugt geeignete Qualität "Poliertonerde P10 feinst" weist eine Agglomeratgröße unter 20 $\mu$m, eine mittlere Primärkristallgröße von 0,5 - 1,5 $\mu$m und ein Schüttgewicht von 500-600 g/l auf.

Als Feuchthaltemittel können Sorbit, Xylit, Glycerin, Propylenglycol oder Gemische dieser Polyole enthalten sein. Auch Polyethylenglycole mit Molekulargewichten von 400 - 2000 können anteilig als Feuchthaltemittelkomponenten enthalten sein. Bevorzugt ist als Feuchthaltemittel Sorbit in einer Menge von 25- 40 Gew.-% enthalten.

Die kondensierten Phosphate sind in Form ihrer Alkalisalze, bevorzugt in Form ihrer Natrium- oder Kaliumsalze enthalten. Die wäßrigen Lösungen dieser Phosphate reagieren aufgrund hydrolytischer Effekte alkalisch. Durch Säurezusatz wird der pH-Wert der erfindungsgemäßen Zahnpflegemittel auf Werte von 7,5 - 9 eingestellt. Als Säuren können dabei z.B. Zitronensäure, Phosphorsäure oder saure Salze, z.B. $NaH_2PO_4$ verwendet werden. Man kann aber auch anteilig saure Salze der kondensierten Phosphate, also z.B. $K_2H_2P_2O_7$ mitverwenden, um den gewünschten pH-Wert des Zahnpflegemittels einzustellen.

Es können auch Gemische verschiedener kondensierter Phosphate oder auch hydratisierte Salze der kondensierten Phosphate eingesetzt werden. Die spezifizierten Mengen von 2 - 12 Gew.-% beziehen sich jedoch auf die wasserfreien Salze. Bevorzugt ist als kondensiertes Phosphat ein Natrium- oder Kalium-Tripolyphosphat in einer Menge von 5 - 10 Gew.-% der Zusammensetzung enthalten.

Eine weitere Verbesserung der Reinigungswirkung der erfindungsgemäßen Zahnreinigungsmittel kann man durch Zusatz eines geeigneten Tensids erzielen. Der Tensidzusatz kann auch zur Erzeugung eines Schaums beim Zähnebürsten, zur Stabilisierung der Poliermitteldispersion sowie zur Emulgierung oder Solubilisierung der Aromaöle erwünscht sein. Geeignete Tenside, die eine gewisse Schaumwirkung entfalten, sind die anionischen Tenside, z.B. Natriumalkylsulfate mit 12 - 18 C-Atomen in der Alkylgruppe. Diese Tenside weisen auch eine gewisse enzymhemmende Wirkung auf, den bakteriellen Stoffwechsel des Zahnbelags. Weitere geeignete Tenside sind Alkalisalze, bevorzugt Natriumsalze von Alkylpolyglycolethersulfat mit 12 - 16 C-Atomen in der linearen Alkylgruppe und 2 - 6 Glycolethergruppen im Molekül, von linearem Alkan-($C_{12}$-$C_{18}$)-sulfonat, von Sulfobernsteinsäuremonoalkyl-($C_{12}$-$C_{18}$)-estern, von sulfatierten Fettsäuremonoglyceriden, sulfatierten Fettsäurealkanolamiden, Sulfoessigsäurealkyl-($C_{12}$-$C_{16}$)-estern, Acylsarcosinen, Acyltauriden und Acylisethionaten mit jeweils 8 -18 C-Atomen in der Acylgruppe.

Auch zwitterionische und ampholytische Tenside können, bevorzugt in Kombination mit anionischen Tensiden, eingesetzt werden. Besonders bevorzugt zur Förderung der Reinigungswirkung ist aber der Einsatz nichtionogener Tenside. Geeignete nichtionische Tenside sind z.B. die Anlagerungsprodukte von Ethylenoxid an Fettalkohole, an Fettsäuren, an Fettsäuremonoglyceride, an Sorbitan-Fettsäuremonoester oder an Methylglucosid-Fettsäuremonoester. Die angelagerte Menge an Ethylenoxid sollte dabei so hoch sein, daß die Tenside wasserlöslich sind, d.h. es sollte wenigstens 1 g/l in Wasser bei 20°C löslich sein. Eine weitere Gruppe von geeigneten Tensiden sind die Alkyl-(oligo)-glycoside mit 8 - 16 C-Atomen in der Alkylgruppe und einem Oligomerisationsgrad des Glycosidrestes von 1 - 4. Alkyl-(oligo)-glycoside, ihre Herstellung und Verwendung als oberflächenaktive Stoffe sind z.B. aus US-A-3,839,318, DE-A-20 36 472, EP-A-77 167 oder WO-A-93/10132 bekannt.

Bezüglich des Glycosidrestes gilt, daß sowohl Monoglycoside (x = 1), bei denen ein Monosaccharidrest glycosidisch an einen Fettalkohol mit 10 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl-(oligo)-glycosid ein Alkyl-(oligo)-glucosid der Formel $RO(C_6H_{10}O)_x$-H, in der R eine Alkylgruppe mit 12 bis 14 C-Atomen ist und x einen Mittelwert von 1 bis 4 hat.

Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäure-sorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von $C_{12}$-$C_{18}$-Fettsäuren und Anlagerungsprodukten von 20 bis 60 Mol Ethylenoxid an 1 Mol Glycerin oder an 1 Mol Sorbit.

Die erfindungsgemäßen Zahnreinigungsmittel enthalten bevorzugt als Lösungsvermittler für gegebenenfalls enthaltene Aromaöle Anlagerungsprodukte von 20 bis 60 Mol Ethylenoxid an gehärtetes oder ungehärtetes Ricinusöl (d.h. an Oxystearinsäure- oder Ricinolsäure-triglycerid), an Glycerin-mono- und/oder -distearat oder an Sorbitanmono- und/oder -distearat.

Als Geschmacksstoffe sind z.B. Süßungsmittel und/oder Aromaöle enthalten. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton.

Darüber hinaus können die erfindungsgemäßen Zahnreinigungsmittel einen therapeutischen Wirkstoff zur Bekämpfung von Karies, Zahnstein, Parodontitis oder anderer Mund- oder Zahnerkrankungen enthalten. Ein bevorzugt enthaltener Wirkstoff ist eine karieshemmende Fluorverbindung, bevorzugt aus der Gruppe der Fluoride oder Monofluorophosphate in einer Menge von 0,1 - 0,5 Gew.-% Fluor. Geeignete Fluorverbindungen sind z.B. Natriumfluorid, Kaliumfluorid, Zinnfluorid, Natriummonofluorophosphat ($Na_2PO_3F$), Kaliummonofluorophosphat oder das Fluorid einer organischen Aminoverbindung.

Weitere geeignete therapeutische Wirkstoffe sind z.B. Antizahnstein-Wirkstoffe, z.B. Organophosphonate wie 1-Azacycloheptan-2,2-diphosphonsäure (Na-Salz) oder 1-Hydroxyethan-1,1-diphosphonsäure (Na-Salz) oder antimikrobielle Plaque-Inhibitoren wie z.B. Hexachlorophen, Chlorhexidin, Hexetidin, Triclosan, Bromchlorophen, Phenylsalicylsäureester. Auch Stoffe, die eine remineralisierungsfördernde und den Verschluß von Dentalläsionen fördernde Wirkung haben, wie z.B. Dicalciumphosphat-dihydrat, bevorzugt in Kombination mit Magnesiumionen, können in den erfindungsgemäßen Zahnpasten enthalten sein.

In einer bevorzugten Ausführungsform enthalten die erfindungsgemäßen Zahnpasten z.B. zusätzlich 1 - 10 Gew.-% Dicalciumphosphat-dihydrat (Brushit) und 0,1 - 0,5 Gew.-% Magnesium-Ionen, bevorzugt in Form eines wasserlöslichen Magnesiumsalzes, z.B. von Magnesiumsulfat, Magnesium-fluorid oder Magnesium-monofluorophosphat.

Schließlich können die erfindungsgemäßen Zahnpasten noch weitere Komponenten enthalten, die in Zahnpflegemitteln üblich sind und die erfindungsgemäßen Effekte nicht verringern. Solche üblichen Zusätze in Zahnpasten sind z.B.:

- weitere Poliermittel in kleineren Mengen von z.B. 1 - 10 Gew.-%, z.B. Calciumcarbonat (Kreide), unlösliches Natriummetaphosphat, Calciumpyrophosphat, Hydroxylapatit, Aluminiumhydroxid, Natriumaluminiumsilikate (Zeolith A) oder teilchenförmige organische Polymere, z.B. Polymethacrylat,
- Pigmente, z.B. Titandioxid oder Zinkoxid,
- Farbstoffe
- pH-Stellmittel und Puffersubstanzen, z.B. Natriumcitrat oder Natriumbicarbonat, Natriumbenzoat,
- wundheilende und entzündungshemmende Stoffe wie z.B. Allantoin, Harnstoff, Panthenol, Azulen oder Kamillenextrakt,

- Konservierungsstoffe wie z.B. Sorbinsäure-Salze, p-Hydroxybenzoesäure-Ester.

Die folgenden Beispiele sollen den Erfindungsgegenstand näher erläutern:

**Beispiele**

Es wurden die folgenden Zahnpasten hergestellt:

| Zusammensetzung | 1 | 2 | 3 | 4 | 5 |
|---|---|---|---|---|---|
| Sident 8 (1) | 14,0 | - | - | - | - |
| Sorbosil AC 39 (2) | - | 14,0 | - | - | 14,0 |
| Zeodent 113 (3) | - | - | 14,0 | - | - |
| Zeodent 623 (4) | - | - | - | 14,0 | - |
| Poliertonerde P10 feinst (5) | 1,0 | 1,0 | 1,0 | 1,0 | 0,5 |
| $Na_5P_3O_{10}$ (Na-Tripolyphoshat) | 5,0 | 5,0 | 5,0 | 5,0 | 5,0 |
| $Na_2PO_3F$ (Na-Monofluorophosphat) | - | 0,8 | - | - | - |
| NaF | 0,24 | - | 0,24 | 0,24 | 0,24 |
| Na-Saccharinat | 0,1 | 0,1 | 0,1 | 0,1 | 0,1 |
| Titandioxid | 0,5 | 0,5 | 0,5 | 0,5 | 0,5 |
| PHB-Methylester | 0,1 | 0,1 | 0,1 | 0,1 | 0,1 |
| Carboxymethylcellulose | 1,25 | 1,25 | 1,25 | 1,25 | 1,25 |
| Sorbit (70 %ig) | 32,0 | 32,0 | 32,0 | 32,0 | 32,0 |
| 1,2-Propylenglycol | 5,0 | 5,0 | 5,0 | 5,0 | 5,0 |
| Cremophor RH 60 (7) | 1,0 | - | 1,0 | 1,0 | 1,0 |
| Arlatone 289 (8) | - | 1,0 | - | - | - |
| Aromaöl | 0,8 | 0,8 | 0,8 | 0,8 | 0,8 |
| Wasser | ad 100 | ad 100 | ad 100 | ad 100 | ad 100 |

| Zusammensetzung | 6 | V1 | V2 | V3 |
|---|---|---|---|---|
| Sorbosil AC 39    (2) | 14 | 14 | 14 | 14 |
| Poliertonerde P 10 feinst (5) | 1 | - | - | - |
| Precarb 100    (6) | - | 2 | 2 | 2 |
| $Na_5P_3O_{10}$ | 10 | 10 | 5 | 0 |
| $Na_2PO_3F$ | 0,8 | 0,8 | 0,8 | 0,8 |
| NaF | - | - | - | - |
| Na-Saccharinat | 0,1 | 0,1 | 0,1 | 0,1 |
| Titandioxid | 0,5 | 0,5 | 0,5 | 0,5 |
| PHB-Methylester | 0,1 | 0,1 | 0,1 | 0,1 |
| Carboxymethylcellulose | 1,25 | 1,25 | 1,25 | 1,25 |
| Sorbit (70 %ig) | 32,0 | 32,0 | 32,0 | 32,0 |
| 1,2-Propylenglycol | 5,0 | 5,0 | 5,0 | 5,0 |
| Arlatone 289   (8) | 1,0 | 1,0 | 1,0 | 1,0 |
| Aromaöl | 0,8 | 0,8 | 0,8 | 0,8 |
| Wasser | ad 100 | ad 100 | ad 100 | ad 100 |

Es wurden die folgenden Handelsprodukte eingesetzt:

(1) Sident®8     : Kieselsäure- Poliermittel

(DEGUSSA)     Mittlere Teilchengröße : 10,0 µm

Siebrückstand 45 µm : ≤ 0,3 %

Stampfdichte     : 300 g/l

(2) Sorbosil AC 39     : Kieselsäure-Poliermittel

(Crosfield Ltd.)     Teilchengröße : 9 – 13 µm

6

(3) Zeodent 113            : Kieselsäure-Poliermittel

    (Huber Chemicals)          Mittlere Teilchengröße : 12 µm

                           Siebrückstand 45 µm    : 1,0 % max.

                           Spez. Oberfläche (BET) : 150 $m^2$/g

(4) Zeodent 623            : Kieselsäure-Poliermittel

    (Huber Chemicals)          Mittlere Teilchengröße : 10 µm

                           Siebrückstand 45 µm    : 0,5 % max.

                           Spez. Oberfläche (BET) : 250 $m^2$/g

(5) Poliertonerde P10 feinst     : Aluminiumoxid-Poliermittel

    (Giulini Chemie)            Mittlere Agglomeratgröße : < 20 µm (min 99 %)

                           Primärteilchen-Größe   : ca. 1 µm

                           Calcinationsgrad : schwach

(6) Precarb 100             : Calciumcarbonat (Kreide)

                           Stampfdichte 0,5 g/ml

                           Spez. Oberfläche : 9.0 $m^2$/g

(7) Cremophor RH 60       : Hydr. Rizinusöl + 60 Mol EO

(8) Arlatone 289           : Hydr. Rizinusöl + 54 Mol EO

                           Schmelzpunkt : 39°C

                           HLB-Wert : 14,4

**Bestimmung der Reinigungswirkung (CRS)**

**Prüfmethode:**

Die Oberfläche von Rinderzähnen wurde konditioniert, unter definierten Bedingungen mit Tee eingefärbt und unter definierten Bedingungen mit der zu prüfenden Zahnpaste gereinigt. Die dabei erzielte Aufhellung wurde farbmetrisch untersucht und mit der Aufhellung verglichen, die mit einer Standard-Prüfpaste erhalten wurde.

**Probenvorbereitung**

Rinder-Schneidezähne wurden in Blöcke von 7 x 7 mm geschnitten und die Blöcke mit Wachs so auf Plexiglasquader (1 x 2,5 x 2,5 cm) montiert, daß nur die Schmelzoberfläche frei blieb. Die Schmelzoberfläche wurde poliert bis sie einheitlich glatt erschien.

**Probenkonditionierung**

Die montierten Zahnblöcke wurden nacheinander in 0,12 N Salzsäure (60 sec.), gesättigte Na$_2$CO$_3$-Lösung (120 sec.) und in 1 %ige Phytinsäure-Lösung (60 sec.) eingetaucht. Nach jeder Behandlung wurden die Proben mit entsalztem Wasser gespült und mit saugfähigem Papier trocken getupft. Die Zahnproben wurden dann in das Anschmutzungsgerät eingehängt und 5 Tage lang durch eine Lösung von schwarzem Tee von 20°C bewegt. Die Teelösung wurde durch Extraktion eines 1,5 g Teebeutels mit 300 g kochenden Wassers (10 Min.) zubereitet und zweimal täglich erneuert.

**Putzversuche**

Die montierten Zahnblöcke wurden dann in eine Grabenstetter V-8-Bürstmaschine eingebracht und in der Zahnpasten-Aufschlämmung aus 20 g Paste und 40 g entsalztem Wasser mit Hilfe einer weichen Oral-B-Zahnbürste mit 150 g Anpreßdruck gebürstet.Als Putz-Standard wurde eine Aufschlämmung von 10 g Calciumpyrophosphat in 50 g einer Carboxymethylcellulose-Quellung (0,5 % CMC, 10 % Glycerin, 89,5 % Wasser) verwendet. Die Reinigungswirkung dieses Standards wird als 100 %-Wert definiert.

**Messung der Reinigungswirkung**

Die Messungen der Aufhellung wurden mit Hilfe eines Dr. Lange Farbdifferenz-Meßgeräts (Typ Micro Color (DC 8334)) gemäß der Deutschen Industrienorm DIN 5033 durchgeführt. Es wurde eine Xenon-Lampe verwendet, die Normallicht D 65, entsprechend dem Tageslicht erzeugt. Als Farbstandard diente Bariumsulfat.

Es wurden zweifache Messungen eines kreisförmigen Bereiches von 7 mm Durchmesser auf der Probenoberfläche durchgeführt. Für jede Zahnpatenprobe wurden 8 angefärbte Zahnproben verwendet und Mittelwerte gebildet. Als Prüfparameter wurde der nach DIN 5033 festgelegte Normfarbwert Y verwendet, der als Maß für die Helligkeit einer Farbe gilt.

Die Helligkeitsmessung erfolgte nach jeweils 1000 Bürsthüben. Die Reinigungswirkung CRS (cleaning ratio soft) ergibt sich aus

$$\text{CRS [\%]} = \frac{\text{mittleres Normfarbwert Y-Increment Test-Paste}}{\text{mittleres Normfarbwert Y-Increment Putzstandard}} \cdot 100 \text{ [\%]}$$

**Bestimmung der Abrasivität (RDA)**

Zur Bestimmung der Abrasivität wurde die RDA-Methodik (radioactive dentin abrasion) nach dem Grabenstetter-Verfahren der Missouri Analytical Laboratories, St. Louis, MO. verwendet (vgl. J. Dent. Res. 17, 1060 - 1068 (1958).

**Ergebnis:**

| Beispiel | 1 | 2 | 3 | 4 | 5 | 6 | V1 | V2 | V3 |
|----------|-----|-----|-----|-----|-----|------|------|-----|------|
| CRS% | 90 | 88 | 103 | 98 | 73 | 97 | 80 | 64 | 31 |
| RDA % | n.b.* | 66 | 55 | 95 | 69 | n.b. | n.b. | 57 | n.b. |

(* n.b. = nicht bestimmt)

Die Ergebnisse zeigen, daß ohne Aluminiumoxid (V1 - V3) trotz hoher Na-Tripolyphosphatgehalte (V1) eine geringere Reinigungskraft erzielt wird, als bei den erfindungsgemäßen Pasten (1 - 4), und daß der Dentinabrieb trotz hoher Reinigungsleistung gegenüber den Vergleichspasten kaum erhöht ist.

**Patentansprüche**

1. Zahnreinigungsmittel in Form einer wäßrigen, pastösen oder flüssigen Dispersion mit einem Gehalt von 10 - 30 Gew.-% einer Poliermittelkombination aus Kieselsäure-Poliermitteln und Aluminiumoxid im Gewichtsverhältnis 10 :

(0,2 - 2) und 20 - 50 Gew.-% eines Feuchthaltemittels aus der Gruppe Sorbit, Glycerin, Propylenglycol-1,2, Polyethylenglycol und Gemischen davon, **dadurch gekennzeichnet, daß** zur Erhöhung der Reinigungswirkung ein kondensiertes Phosphat aus der Gruppe Tripolyphosphat, Pyrophosphat oder Trimetaphosphat in Form eines Alkali- oder Ammoniumsalzes in einer Menge von 2 - 12 Gew.-% enthalten ist.

2. Zahnreinigungsmittel nach Anspruch 1, **dadurch gekennzeichnet, daß** als Aluminiumoxid eine schwach calcinierte Tonerde mit einem Gehalt von wenigstens 10 Gew.-% von Alpha-Aluminiumoxid verschiedener $Al_2O_3$-Modifikationen enthalten ist.

3. Zahnreinigungsmittel nach einem der Ansprüche 1 oder 2, **dadurch gekennzeichnet, daß** als Feuchthaltemittel Sorbit in einer Menge von 25 - 40 Gew.-% enthalten ist.

4. Zahnreinigungsmittel nach einem der Ansprüche 1 - 3, **dadurch gekennzeichnet, daß** als kondensiertes Phosphat 5 - 10 Gew.-% Natrium- oder Kalium-Tripolyphosphat enthalten ist.

5. Zahnreinigungsmittel nach einem der Ansprüche 1 - 4, **dadurch gekennzeichnet, daß** als Kieselsäurepoliermittel eine Fällungskieselsäure mit einer Teilchengröße von 5 - 20 μm und einer spezifischen Oberfläche (BET) von 100 - 300 g/l enthalten ist.

6. Zahnreinigungsmittel nach einem der Ansprüche 1 - 5, **dadurch gekennzeichnet, daß** eine Fluorverbindung, bevorzugt aus der Gruppe der Fluoride oder Monofluorophosphate in einer Menge von 0,1 - 0,5 Gew.-% Fluor enthalten ist.

**Claims**

1. Dentifrices in the form of water-containing, paste-form or liquid dispersions containing 10 to 30% by weight of a combination of silica polishing agents and aluminium oxide in a ratio by weight of 10:(0.2-2) and 20 to 50% by weight of a humectant from the group consisting of sorbitol, glycerol, 1,2-propylene glycol, Polyethylene Glycol or mixtures thereof, **characterized in that** they contain a condensed phosphate from the group consisting of tripolyphosphate, pyrophosphate and trimetaphosphate in the form of an alkali metal or ammonium salt in a quantity of 2 to 12% by weight in order to increase the cleaning effect.

2. A dentifrice as claimed in claim 1, **characterized in that** lightly calcined alumina containing at least 10% by weight of α-aluminium oxide of various $Al_2O_3$ modifications is present as the aluminium oxide.

3. A dentifrice as claimed in claim 1 or 2, **characterized in that** sorbitol is present in a quantity of 25 to 40% by weight as humectant.

4. A dentifrice as claimed in any of claims 1 to 3, **characterized in that** 5 to 10% by weight of sodium or potassium tripolyphosphate is present as the condensed phosphate.

5. A dentifrice as claimed in any of claims I to 4, **characterized in that** a precipitated silica with a particle size of 5 to 20μm and a specific surface (BET) of 100 to 300 g/l is present as the silica polishing agent.

6. A dentifrice as claimed in any of claims 1 to 5, **characterized in that** a fluorine compound, preferably from the group of fluorides or monofluorophosphates, is present in a quantity of 0.1 to 0.5% by weight flourine.

**Revendications**

1. Agent nettoyant à usage dentaire sous forme de dispersion aqueuse, pâteuse ou liquide avec une teneur de 10-30% en poids d'une combinaison d'agents polissants constituée d'agents polissants à base d'acide silicique et d'oxyde d'aluminium dans un rapport en poids de 10:(0,2-2) et de 20-50% en poids d'un agent humectant sélectionné parmi le groupe des sorbitol, glycérine, propylèneglycol-1,2, polyéthylèneglycol et des mélanges de ceux-ci, **caractérisé en ce qu'**il contient, pour augmenter l'action nettoyante, un phosphate condensé sélectionné parmi le groupe des tripolyphosphate, pyrophosphate ou trimétaphosphate, sous la forme d'un sel alcalin ou d'ammonium dans une quantité de 2-12% en poids.

**2.** Agent nettoyant à usage dentaire selon la revendication 1, **caractérisé en ce qu'**il contient comme oxyde d'aluminium de l'alumine légèrement calcinée avec une teneur d'au moins 10% en poids d'oxyde d'aluminium alpha de différentes modifications d'$Al_2O_3$.

**3.** Agent nettoyant à usage dentaire selon l'une des revendications 1 ou 2, **caractérisé en ce qu'**il contient comme agent humectant du sorbitol dans une quantité de 25-40% en poids.

**4.** Agent nettoyant à usage dentaire selon l'une des revendications 1-3, **caractérisé en ce qu'**il contient comme phosphate condensé 5-10% en poids de tripolyphosphate de sodium ou de potassium.

**5.** Agent nettoyant à usage dentaire selon l'une des revendications 1-4, **caractérisé en ce qu'**il contient comme agent polissant d'acide silicique un acide silicique de précipitation d'une grosseur de particules de 5-20 $\mu$m et d'une surface spécifique (BET) de 100-300 g/l.

**6.** Agent nettoyant à usage dentaire selon l'une des revendications 1-5, **caractérisé en ce qu'**il contient un composé de fluor, de préférence sélectionné parmi le groupe des fluorures ou des monofluorophosphates dans une quantité de 0,1-0,5% en poids de fluor.